(19) Europäisches Patentamt
European Patent Office
Office européen des brevets

(11) Publication number: **0 162 413**
**A2**

(12) # EUROPEAN PATENT APPLICATION

(21) Application number: 85105980.8

(22) Date of filing: 15.05.85

(51) Int. Cl.[4]: A 61 K 31/13
//(A61K31/13, 31:195, 31:215, 31:13, 45:02)

(30) Priority: 17.05.84 US 611225
04.04.85 US 718483

(43) Date of publication of application:
27.11.85 Bulletin 85/48

(84) Designated Contracting States:
AT BE CH DE FR GB IT LI LU NL SE

(71) Applicant: MERRELL DOW PHARMACEUTICALS INC.
2110 E. Galbraith Road
Cincinnati Ohio 45215(US)

(72) Inventor: Prakash, Nellikunja J.
10871 Wengate Lane
Cincinnati Ohio 45241(US)

(72) Inventor: Sunkara, Sai P.
9629 Linfield Drive
Cincinnati Ohio 45242(US)

(72) Inventor: Stemerick, David M.
11433 Kentbrook Court
Cincinnati Ohio 45240(US)

(72) Inventor: Edwards, Michael L.
12033 Deerhorn Drive
Cincinnati Ohio 45240(US)

(74) Representative: Sgarbi, Renato et al,
GRUPPO LEPETIT S.p.A. Patent and Trademark
Department Via Roberto Lepetit, 8
I-20124 Milano(IT)

(54) **Polyamine for use as a medicine in the treatment of neoplasms.**

(57) This invention relates to the use of certain polyamines in the treatment of neoplasms or in conjunctive therapy with ornithine decarboxylase inhibitors and/or S-adenosylmethionine decarboxylase inhibitors or in conjunction with other known cytotoxic agents, or with interferon.

EP 0 162 413 A2

Croydon Printing Company Ltd

# POLYAMINE FOR USE AS A MEDICINE IN THE TREATMENT OF NEOPLASMS.

This invention relates to certain polyamines for use as a medicine. In particular, this invention relates to certain polyamine for use in the treatment of neoplasms, either alone or in conjunctive therapy with ornithine decarboxylase inhibitors and/or S-adenosylmethionine decarboxylase inhibitors or in conjuntion with other known cytotoxic agents, or with Interferon.
More specifically, this invention relates to the treatment of neoplasms with polyamines of the formula

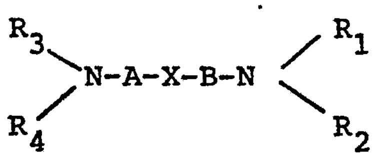

I

and the pharmaceutically acceptable acid addition salts thereof,
wherein X is $-CH_2-$, NR, O, S, SO or $SO_2$,
R is H, $C_{1-6}$ lower alkyl or benzyl, each of
$R_1$ and $R_3$ independently are H, $C_{1-6}$ lower alkyl,
$-(CH_2)_3NH_2$,
$-\overset{O}{\overset{\|}{C}}-C_{1-6}$alkyl, or $-\overset{O}{\overset{\|}{C}}-$phenyl, each of
$R_2$ and $R_4$ independently are H or $C_{1-6}$ lower alkyl,
A is a $C_{3-4}$ lower alkylene moiety optionally substituted with a $C_{1-4}$ lower alkyl moiety radical, and
B is a $C_{2-4}$ lower alkylene moiety optionally substituted with a $C_{1-4}$ lower alkyl radical.

In the definition of the compounds of formula I, the $(C_1-C_6)$alkyl radical includes straight or branched chain saturated aliphatic hydrocarbyl radicals having up to six carbon atoms, with methyl, ethyl and hexyl being preferred. The alkylene moieties of the A and B designations include the ethylene [$-(CH_2)_2-$], propylene [$-(CH_2)_3-$] and butylene [$-(CH_2)_4-$] straight chain radicals each of which may also be substituted with a methyl, ethyl, propyl or butyl radical. When X is $-CH_2-$ then the combination of A, X and B moieties represent a straight chain alkylene having 6-9 carbon atoms optionally (but not preferentially) bearing one or two $(C_1-C_4)$alkyl radicals. Included within the NR definition are the mono and di-substituted N-benzyl compounds wherein the substituents are hydroxy, halogen, $(C_1-C_4)$alkyl, and $(C_1-C_4)$alkoxy. The 3',4'-dihydroxy and the 3',4'-dimethoxy substituted benzyl moieties being preferred. The term "halogen" is intended to encompass chloro, bromo, iodo and fluoro atoms. In practice, it is preferred to employ those compounds of Formula I wherein all of $R_1$, $R_2$, $R_3$ or $R_4$ are hydrogen or wherein $R_1$ and $R_3$ are aminopropyl and $R_2$ and $R_4$ are hydrogen. Another preference is when A and B are unsubstituted. Still another preference is when A and B each represent a propylene moiety. A further preference when A and B are other than propylene in that B represents an alkylene moiety having one less carbon atom than the alkylene moiety for A. Still another preference is that X represent either $-CH_2-$ or NR. When X is $CH_2$ it is preferred that the combined moieties of A, X and B represent a straight-chain alkylene moiety having 8 carbon atoms, preferentially neither A or B bearing any lower alkyl substituents. When X is other than $CH_2$, the

preferred X moiety is NH, wherein, in that instance, the preferred compound is norspermidine, a compound wherein X is NH, A and B is an unsubstituted propylene moiety and each of $R_1$, $R_2$, $R_3$ and $R_4$ is hydrogen. Still another preference is when norspermidine is substituted such that either or both of $R_1$ and $R_3$ are aminopropyl. The most preferred compounds are those wherein X is $CH_2$ with $R_1$ and $R_3$ being aminopropyl. The most preferred compound being N,N'-bis ( 3-aminopropyl)-1,8-octanediamine.

In practice, the compounds are believed to be useful in the treatment of neoplasms which are dependent upon polyamine biosynthesis for their growth. Such neoplasms include leukemia, including but not limited to acute lymphoblastic, chronic lymphocytic, acute myloblastic and chronic mylocytic; Carcinomas, including but not limited to those of the cervix, oesphagus, stomach, small intestines, colon and lungs; Sarcomas, including but not limited to oesteroma, osteosarcoma, lepoma, liposarcoma, hemangioma and hemangiosarcoma; Adenomas, both benign and malignant, including, for example, the thyroid, parathyroid, prostate and the like; Melanomas, including amelonotic and melanotic; and mixed types of neoplasias such as, for example, adenocarcinoma, carcinosarcoma, lymphoid tissue type, folicullar reticulum, cell sarcoma and Hodgkins Disease.

In effecting treatment of the neoplastic disease state, it is preferred to administer a compound of formula I to a patient suffering from a neoplasm with about 15-100 mg per kilogram of body weight per day. The specific

amount depends upon the severity of the state of the neoplasm such as may be determined by the attending diagnostician. In practice, the ideal is to deprive the diseased cell of its opportunity for proliferative cell growth by inhibiting polyamine biosynthesis. Thus, in the practice of this invention, it is preferred that a sufficient blood level of the polyamine of formula I be maintained so as to inhibit polyamine biosynthesis; and, more specifically, to inhibit the formation of putrescine and spermidine. The precise dosage required to maintain the desired blood level of the polyamine of formula I to inhibit polyamine biosynthesis (specifically inhibition of putrescine and spermidine) may be determined by the use of standard pharmaco-kinetic studies well established for these determinations. Of course the compounds of this invention may be administered to a patient orally, intravenously, intraperitoneally and/or subcutaneously, preferably orally. In any case, the pharmaceutical preparations may be effected by standard techniques well known in the art.

The term "treatment of neoplasms" is meant to include treating the patient having neoplasms so that the therapy has an advantageous effect by "controlling the growth" of neoplasm, which for the purposes of this invention includes slowing, interrupting, arresting or stopping the growth and metastases of the proliferating tumor tissue in a warm blooded animal; it being understood that such does not necessarily provide for a "cure" for the tumor in the sense that the tumor tissue is totally eliminated.

Another aspect of this invention is to utilize he compounds of this invention with ornithine decarboxylase inhibitors (ODC inhibitors) and/or S-adenosylmethionine

decarboxylase inhibitors (SAM-DC inhibitors). Although any of the functional irreversible ODC inhibitors may be utilized in conjunctive therapy with the compounds of formula I, compounds such as methyl acetylenic putrescine and compounds of the formulae

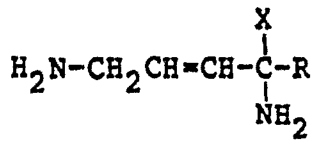

II

and

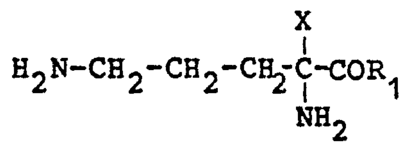

III

wherein X is $-CHF_2$ or $-CH_2F$,
R is H or $COR_1$,
$R_1$ is OH or $C_{1-6}$ alkoxy,
and the pharmaceutically acceptable salts and individual optical isomers thereof are preferred. Particularly preferred compounds are α-difluoromethyl ornithine (α-DFMO), the methyl and ethyl esters of monofluoromethyl dehydroonithine, and the R,R-isomer of methyl acetylenic putrescine (i.e., (2R, 5R)-6-heptyne-2,5-diamine).

Other agents with which the compounds of this invention may be utilized in conjunctive therapy are cyclophosphamide, methotrexate, prednisone, 6-mercaptopurine, chlorambucil, cytosine arabinoside, 6-thioguanine, thio TEPA, 5-fluorouracil, 5-fluoro-2-deoxyuridine, 5-azacytidine, nitrogen mustard, 1,3-bis(2-chloroethyl)-1-nitrosourea (BCNU), (1-(2-chloroethyl)-3-cyclohexyl-1-nitrosourea) (CCNU), busulfan, adriamycin, bleomycin, vindesine, cycloleucine or methylglyoxal bis(guanylhydrazone) (i.e., MGBG).

In a preferred manner, a compound of this invention can be administered with an ODC or SAM-DC inhibitor, or a known cytotoxic agent known to be useful in tumor therapy. When such combination therapy is employed for the treatment of a tumor, the cytotoxic agent may be administered at a dosage known in the art to be effective for treating the tumor. However, the combination with an ODC inhibitor may produce an additive or synergistic effect with the compounds of formula I or with a cytotoxic agent against a particular tumor. Thus, when such combination antitumor therapy is used, the dosage of the cytotoxic agent administered may be less than that administered when the cytotoxic agent is used alone. In combination with an ODC inhibitor, the compounds of this invention or the cytotoxic agent may, therefore, be administered at a lower dosage level or at less frequent intervals as compared to the cytotoxic agent when used alone. Similarly, when a compound of this invention is used with an ODC inhibitor, alone or in combination with a cytotoxic or other herein mentioned therapeutic agent, the dosage of the ODC inhibitor or other conjunctively utilized agent, may be less then when used without a compound of this invention (i.e., the compound of formula I).

The term "combination therapy" or "conjunctive therapy" refers to the simultaneous, separate or sequential use of a preparation in the specified treatment. "Combination" and "combination therapy", "conjunction" and "conjunction therapy" are therefore held to encompass also the case where the active components of the combination or conjunction are parts of a medical kit, collection or package containing them in separate form or their existence as a medical kit collection of package, although as such not immediately evident, become evident in view of the indication of purpose for

the combined therapy. The term "combination therapy" or "conjunctive therapy" contemplates therefore, for example, the administration of an ODC inhibitor immediately prior to the beginning of therapy with a compound of this invention and a cytotoxic agent, concomitantly with such therapy, or during the period of time immediately following cessation of such therapy. Preferably, the patient is treated with an ODC inhibitor for about 1 to 14 days, preferably 4 to 14 days, prior to the beginning of therapy with a cytotoxic agent, and thereafter, on a daily basis during the course of such therapy. Daily treatment with the ODC inhibitor can be continued for a period of, for example, 1 to 365 days after the last dose of the cytotoxic agent is administered.

As pharmacologically useful agents, the compounds of this invention can be administered in various ways to the patient being treated to achieve the desired effect. The compounds can be administered either alone or in combination with one another, or they can be administered with the other aforementioned compounds of this invention in the form of pharmaceutical compositions, the preparation of which are well known in the art. The compounds may be administered orally or parenterally (for example, intravenously, intraperitoneally or subcutaneously), including injection of the active ingredient directly into the tumor. The amount of compound administered will vary over a wide range and can be any effective amount. Depending upon the patient to be treated, the severity of the condition being treated, the mode of administration, and the particular compound employed, the effective amount of compound of this invention (I) administered will vary from about 15 mg/Kg to 100 mg/Kg

of body weight of the patient per day and preferably will be about 25 mg/Kg to 50 mg/Kg of body weight of patient per day.

The solid unit dosage forms can be of the conventional type. Thus, the solid form can be a capsule which can be of the ordinary gelatin type containing a novel compound of this invention and a carrier, for example, lubricant and inert fillers, such as lactose, sucrose or corn starch in combination with binders such as acacia, corn starch or gelatin, desintegrating agents such as corn starch, potato starch or alginic acid, and a lubricant such as stearic acid or magnesium stearate.

For parenteral administration, the compounds may be administered as injectable dosages of a solution or suspension of the compound in a physiologically acceptable diluent with a pharmaceutical carrier which can be a sterile liquid such a water and oils with or without the addition of a surfactant and other pharmaceutically acceptable adjuvants. Illustrative of oils which can be employed in these preparations are those of petroleum, animal, vegetable to synthetic origin, for example, peanut oil, soyben oil and mineral oil. In general, water, saline, aqueous dextrose and related sugar solutions, ethanols and glycols such as propylene glycol or polyethylene glycol are preferred liquid carriers, particularly for injectable solutions.

The results in the treatment of rapidly proliferating tumor tissue disease states when used in combination with compounds of this invention have demonstrated a striking tumor supression and such results can be shown and ascertained by standard and well-known laboratory techniques as may be illustrated by the following.

TABLE I

Effect of Norspermidine On The Growth and Polyamine Levels of HeLa Cells In Culture*

| | | | | Polyamine Concentration (n·moles/$10^6$ cells) | | | |
|---|---|---|---|---|---|---|---|
| Treatment | Cell No. x $10^6$ | % Inhibition of Cell Growth | Mitotic Index | Norspermidine | Putriscine | Spermidine | Spermine |
| Control | 1.80 | -- | 5.8 | 0 | 0.840 | 3.08 | 2.35 |
| Norspermidine | | | | | | | |
| 0.5 mM | 0.70 | 61 | 71.0 | 6.32 | ND | 0.481 | 0.441 |
| 0.05 mM | 0.64 | 65 | 76.7 | 4.30 | ND | 0.433 | 0.452 |
| 0.005 mM | 0.70 | 61 | 74.0 | 5.31 | 0.026 | 0.849 | 0.637 |

*HeLa cells were plated at a density of 0.5 x $10^6$/100 mm dish an incubated overnight. Different concentrations of norspermidine were added and cell growth and mitotic index were determined after 72 hours of incubation at 37°C. A portion of the cells were used for polyamine determinations.

ND: not detectable.

TABLE 2

Effect of Norspermidine On the Growth And Polyamine Levels of L1210 Leukemia in Mice

| Treatment | Survival (Days) | % T/C | Polyamine Concentration (n-moles/$10^6$ cells) Putrescine | Spermidine | Spermine |
|---|---|---|---|---|---|
| Control | 7.5 | -- | 0.17 | 1.77 | 0.85 |
| Norspermidine | 11.0 | 147 | ND | 0.69 | 0.77 |
| DFMO | 8.0 | 107 | 0.015 | 0.84 | 1.18 |
| DFMO and Norspermidine | 14.0 | 187 | ND | 0.29 | 0.84 |

1 x $10^5$ L1210 leukemic cells per animal were innoculated i.p. on day 0 and drug treatments were started on day 1. DFMO was administered as 2% solution in drinking water (approximately 3 g/kg based on fluid intake). Norspermidine was administered i.p. at 50 mg/kg/day from day 1 to 7. Polyamine concentrations were determined on cells collected from treated and control animals on day 4. ND: not detectable;

$$\% \ T/C : \ 100 \times \frac{\text{survival (days) in treated}}{\text{survival in control}}$$

TABLE 3

Effect of DFMO and Norspermidine On The Growth of Lewis Lung Tumor (3LL) in Mice

| Treatment | Tumor Weight (gr) | % Inhibition |
|---|---|---|
| Control | 5.15 | - |
| Norspermidine | 3.50 | 32 |
| DFMO | 4.92 | 4 |
| DFMO and Norspermidine | 0.86 | 83 |

1 x $10^5$ 3LL cells per animal were injected s.c. in the intrascapular region of the mice on day 0. Drug treatments were started on day 1. DFMO was administered as a 2% solution in drinking water (approximately 3 g/kg based on fluid intake). Norspermidine was administered i.p. 50 mg/kg/day from day 1 to 14. At the end of 18 days, animals were sacrificed, tumors disected and weighed.

TABLE 4

Effect of DFMO and Norspermidine On The Survival of $B_{16}$ Melanoma Bearing Mice

| Treatment | Survival (Days) | % T/C |
|---|---|---|
| Control | 13.5 | - |
| Norspermidine | 18.0 | 133 |
| DFMO | 17.5 | 129 |
| DFMO and Norspermidine | 23.0 | 170 |

*DFMO = α-difluoromethyl ornithine

1 x $10^5$ $B_{16}$ melanoma cells per animal were injected i.p. on day 0. Drug treatments were started on day 1. DFMO was administered as a 2% solution in drinking water (approximately 3 g/kg based on fluid intake). Norspermidine was administered i.p. at 50 mg/kg/day starting day 1 to 7.

% T/C: $$100 \times \frac{\text{survival in treated}}{\text{survival in control}}$$

TABLE 5

Effect Of Norspermidine On the Polyamine Biosynthetic Enzymes of L1210 Leukemia In Mice

| | Ornithine Decarboxylase | | S-Adenosylmethiamine Decarboxylase | |
|---|---|---|---|---|
| Treatment | (cpm/mg protein) | % Inh. | (cpm/mg protein) | % Inh. |
| Control | 4780 | -- | 1060 | -- |
| Norspermidine | 150 | 99.9% | 590 | 45 |

1 x $10^5$ leukemia cells per animal were injected on day 0. Nospermidine was injected on day 3 at 50 mg/kg. The cells were collected on day 4 from animals, washed and used for enzymatic determination.

## TABLE 6

Effect Of N,N'-bis(3-aminopropyl)-1,8-octanediamine on the Growth of Human Cervical Cancer (HeLa) And Mouse Melanoma ($B_{16}$) Cells In Culture

| Treatment | HeLa Cells Cell No. x $10^5$ | HeLa Cells % Inh. of Cell Growth | $B_{16}$ Melanoma Cell No. x $10^6$ | $B_{16}$ Melanoma % Inh. of Cell Growth |
|---|---|---|---|---|
| Control | 11.9 | -- | 17.3 | -- |
| Compound | | | | |
| 1.0 mM | 2.82 | 76 | 0 | 100 |
| 0.1 mM | 4.56 | 62 | 7.42 | 57 |
| 0.01 mM | 5.9 | 50 | 7.40 | 57 |
| 0.001 mM | 7.56 | 39 | 14.5 | 16 |

HeLa cells and $B_{16}$ melanoma cells were plated at a density of 1 x $10_5$/plate (35 mm) and incubated overnight. Different concentrations of the compound in regular medium were added and cell growth was determined after 72 hours of incubation at 37°C.

TABLE 7

AntiTumor Activity Of N,N'-bis(3-aminopropyl)-1,8-octanediamine Against L1210 Leukemia In Mice

| Treatment | Survival (Days) | % T/C |
|---|---|---|
| Control | 7.5 ± 0.5 | |
| Compound | | |
| 25 mg/kg* | 16.0 ± 1.5 | 213.3 |
| 6.26 mg/kg** | 27 ± 3.0 | 360.0 |

1 x $10^5$ L1210 leukemic cells/animal were inoculated i.p. on day 0 and drug treatments were started as follows:

* N,N'-bis(3-aminopropyl)-1,8-octanediamine was administered i.p. at 25 mg/Kg/day from day 1-7.

** The compound was administered i.p. at 6.25 mg/kg 4 times a day, every three hours starting at 8:30 a.m. to 5:30 p.m. from days 3-7.

$$\% \ T/C = 100 \times \frac{\text{survival (days) in treated}}{\text{survival in control}}$$

TABLE 8

Antitumor activity of N,N'-bis(3-aminopropyl)-1,8-octanediamine against $B_{16}$ Melanoma and Lewis Lung carcinoma in mice.

| Treatment | $B_{16}$ Melanoma Tumor Weight (gms) | % Inh. | Lewis Lung Carcinoma Tumor Weight (gms) | % Inh. |
|---|---|---|---|---|
| Control | $4.90 \pm 0.39$ | -- | $4.95 \pm 0.37$ | -- |
| Compound | | | | |
| 6.25 mg/kg | $1.68 \pm 0.37$ | 66 | $3.10 \pm 0.39$ | 37 |
| 12.5 mg/kg | $0.95 \pm 0.29$ | 81 | $1.94 \pm 0.24$ | 61 |

$1 \times 10^5$ tumor cells/animal were inoculated s.c. on day 0 and the drug treatments were started on day 1. N,N'-bis(3-aminopropyl)-1,8-octanediamine was administered at different doses from day 1-10. The animals were sacrificed on day 15 and the tumors were dissected and weighed.

Still another aspect of this invention is the treatment of the hereinabove mentioned neoplastic disease states utilizing a polyamine of formula I in conjunctive therapy with Interferon; this combination may be alone or in a further combination with an irreversible ODC inhibitor and/or known cytotoxic agents.

The preferred ODC inhibitors again are α-monofluoromethyl ornithine, α-difluoromethyl ornithine, the methyl and ethyl esters of monofluoromethyl

dihydroornithine and the R,R-isomer of methyl acetylenic putrescine although any of the above defined ODC inhibitors of formula I and I may be used.

According to this aspect of the invention the term "Interferon" is used in its generic meaning. Although known for over twenty years, Interferon has not yet been sufficiently characterized as to be subject to a precise definition and thus some difficulty arises in the use of the term. However, it is a term that is attached to those glycoproteins which are made by all animal cells after viral attack, and which function as regulating functionaries of the immune system. Interferon has also been made biosynthetically by recombinant DNA techniques and thus the precise characterization and/or nomenclature of such substance has been further complicated. However, as used in the art and in the context of this application, the term Interferon is meant to include all those natural and biosynthetic interferons capable of immunomodulation and include Type I and Type II interferons as well as the recombinant DNA types. Type I interferons (also sometimes called leucocyte interferons) include those α and β interferons generally prepared from leucocytes by viral infection. Type II interferons, sometimes called immunoactive interferon (α interferon) are those which are generally prepared from lymphocytic reactions with mitogenes. Although, as stated above, the recombinant interferons are included herein, the immune and leucocyte interferons are preferred. Aso included within the concept of the present invention is the embodiment of the treatment of tumor tissue with ODC inhibitors and Interferon when the Interferon is generated by the use of Interferon inducers (e.g., tilorone and its related analogs known a interferon inducers) which, in situ, induce the release of Interferon.

A still further aspect of this invention is the use of a polyamine of formula I and Interferon in conjunctive therapy with known non-steroidal antiandrogenic agents (e.g., Flutamide and the like), with or without the added effects of ODC inhibitors, in the treatment of androgen-dependent disease states such as benign prostatic hypertrophy, prostatic carcinoma, mammary carcinoma and other androgen-dependent tumor sites throughout the body. When a polyamine of formula I is used with Interferon in combination with an ODC inhibitor in conjunction with a cytotoxic agent most known cytotoxic agents may be employed. Illustrative examples of cytotoxic agents are cyclophosphamide, methotrexate, prednisone, 6-mercaptopurine, procarbazine, daunorubicin, vincristine, cinblastine, chlorambucil, cytosine arabinoside, 6-thioguanine, thio TEPA, 5-fluorouracil, 5-fluoro-2-deoxyuridine, 5-azacytidine, nitrogen mustard, 1,3-bis(2-chloroethyl)-1-nitrosourea (BCNU), (1-(2-chloroethyl)-3-cyclohexyl-1-nitrosourea) (CCNU), busulfan, adriamycin, bleomycin, bindesine, cycloleucine or methylglyoxal bis(guanylhydrazone) (i.e., MGBG).

For the most part, the compounds of formula I are known compounds. Those few which are novel may readily by prepared by analogous techniques well known to those in the art.

More specifically, those compounds of formula I wherein any of the R groups are hydrogen are known or else may be made by analogous and routine techniques. Preparation of the amido, acyl, aroyl, alkyl and benzyl derivatives may readily be effected by standard techniques. For example, the N-alkylation or N-benzylation of the central nitrogen atom (i.e., wherein X

is NH) is effected by first forming a Schiff's base at the nitrogen atoms attached to the "A" and "B" alkylene moieties (these nitrogen atoms will hereinafter be referred to as "terminal" nitrogen atoms). The formation of the Schiff's bases is effected by reaction of the polyamine with an aldehyde (preferably benzaldehyde) in an inert organic solvent (e.g., methylene chloride) in the presence of a molecular sieve. Following the formation of the Schiff bases, the central hydrogen atom is alkylated or benzylated by forming an anion with sodium hydride and the resulting anion is reacted with the appropriate alkyl or benzyl halide. The Schiff bases are then converted to the amines by hydrolysis in dilute aqueous acid according to standard techniques.

To form the amides (i.e., those compounds wherein $R_1$ and $R_3$ contain the acyl or aroyl moiety), the appropriate intermediate is reacted with an acyl or aroyl halide or anhydride, using standard amidation techniques, taking the usual step of first protecting the center nitrogen atom according to standard techniques. Following the amidation, the N-protecting group is removed according to standard and well-known techniques.

The compounds of formula I wherein X is oxygen are readily prepared from a terminal amide - terminal nitrile alkyl ether (IV) which are prepared by the action of a sodium alkoxide of the appropriate haloalkyl nitrile in an inert organic solvent (e.g., THF) according to the following reaction scheme:

$$R'\overset{O}{\overset{\|}{C}}-\overset{H}{N}-(CH_2)_mO^{\ominus}Na \quad + \quad X(CH_2)_{n-1}CN \longrightarrow$$

II III

$$R'\overset{O}{\overset{\|}{C}}-\overset{H}{N}-(CH_2)_mO(CH_2)_{n-1}CN$$

IV

wherein R' is lower alkyl or phenyl, m is 4 or 3 and n is 4, 3 or 2. Compounds of IV are chemically reduced by the action of hydrogen in the presence of an Adams catalyst, $PtO_2$ and an alkyl or aroyl anhydride or mixed anhydride to yield amides of the formula

$$R'-\overset{O}{\overset{\|}{C}}-\overset{H}{N}(CH_2)_mO(CH_2)_n\overset{H}{N}-\overset{O}{\overset{\|}{C}}-R'$$

V

Hydrolysis with refluxing 6N HCl yields the bis unsubstituted amino compounds. Lithium aluminum hydride reduction of the amide (V) yields the bis-N-alkylated or N-benzylated compounds. Should the tetra alkyl derivatives (i.e., the bis dialkyl derivatives) be desired, the sequential acylation - reduction procedures may be repeated on the dialkyl derivatives.

Those compounds for formula I wherein X is S are prepared by the Michael addition reaction of an amino substituted thiol with acrylonitrile to yield the desired amino nitrile (e.g., $H_2$NASBCN) according to standard conditions and techniques. Standard reduction procedures (e.g., using $LiAlH_4$ or catalytic hydrogenation) are utilized to convert the nitrile to the desired compounds

of formula I (i.e., those wherein X is S, and $R_1$, $R_2$, $R_3$ and $R_4$ are hydrogen). Oxidation of these thioether compounds to the corresponding sulfoxide is effected by reaction of the thioether with sodium metaperiodate in water or aqueous ethanol according to standard techniques. Further oxidation to the sulfone (i.e., X is $SO_2$) is effected with a peracid (e.g., meta chlorobenzoic acid) in an organic solvent (e.g., chloroform or dichloromethane). The sulfone may also be prepared from the thioether using two equivalents of the peracid. As previously described, the amines may be acylated by reaction with an acyl halide or acid anhydride under standard conditions. The resulting bis-amide may be reduced with $LiAlH_4$ to yield the dialkyl derivatives. Should the tetra alkyl derivatives (i.e., the bis dialkyl derivatives) be desired, the sequential acylation - reduction procedures may be repeated on the dialkyl derivatives.

Conversion of the compounds of formula I can be converted to their pharmaceutically acceptable salts using standard techniques (e.g., neutralizing with hydrochloric acid).

Although, in general, the compounds of formula I are already known in the art, for following examples typify their preparation.

## EXAMPLE I

N,N'-bis (3-aminopropyl)-1,8-octanediamine 4 HCl

Step A: 1,8-Bis cyanoethyl diaminooctane:

Combine 1,8-diamine octane (14.4 g - 0.1 mole) and 200 ml anhydrous ethanol, add acrylonitrile (11.4 g/14.4 ml - 0.2 mole) and reflux the mixture with stirring for 18 hours. Remove the solvent at reduced pressure to yield,

after short path distillation, 14.3 of the desired intermediate, bp. 152°C/0.1 mm.

Step B: Treat a mixture of the dinitrile of Step A, 1.0 G of platinum oxide, 150 ml acetic acid and 28.5 ml of 12N HCl with hydrogen at 45 lbs/in.$^2$ until no further hydrogen is taken up; filter the resulting mixture and remove the solvent under reduced pressure. Add three 100 ml portions of ethanol and remove at reduced pressure. recrystallize white solid from 1 liter of 10% aqueous ethanol to obtain 9.0 gm of desired product mp >300°c.

EXAMPLE 2

3-[(2-Ethylamino)ethyl]thiopropylamine

Step A: acetamidoethyl-2-cyanoethylsulfide:

Sodium hydride (10 mg of 50% mineral oil dispersion, 0.2 mmole) is added to a solution of N-(-2meraptoethyl)-acetamide (1.35g, 0.01 mole) and acrylonitrile (1.06 g, 0.02 mole) in tetrahydrofuran (50 ml). The reaction is stirred at ambient temperature until TLC indicates complete reaction. The reaction mixture is treated with 2 ml of methanol to destroy any unreacted NaH and poured into 100 ml of ice-water. The aqueous mixture is extracted twice with 100 ml portions of ethylacetate. The combined organic extracts are washed with 1N NaOH, dried and evaporated. The title compound of this step is used in Step B without further purification.

Step B: 3-[(2-Ethylamino)Ethyl]thiopropylamine:

2-Acetamidoethyl-2-cyanoethylsulfide (1.62 g, 0.01 mole) is dissolved in anhydrous ether (100 ml) and the solution is added dropwise to a suspension of lithium aluminum hydride (1 g, 0,025 mole) in ether (400 ml). The mixture is stirred until TLC indicates complete reaction. The excess hydride is decomposed with aq. KOH and the mixture is continuously extracted with ether for 48 hours.

The ether extracts are dried with KOH and decanted. The dried extract is treated with excess etheral HCl to give the dihydrochloride of the desired product.

EXAMPLE 3

3,3'-Thio-bis-1-propanamine

Reduction of 3-[(2-cyanoethyl)thio]propylamine (prepared by method in Netherlands Patent 6,507,298) by the manner described in Step B of Example 2 yields the dihydrochloride of 3,3'-thio-bis-1-propanamine.

EXAMPLE 4

3,3'-Thio-bis-1-propanamine sulfoxide

3,3'-Thio-bis-1-propanamine (670 mg, 5 mmole) is dissolved in a mixture of water (50 ml) and ethanol (5 ml). Sodium metaperiodate (1.17 g, 6 mmole) is added and the mixture is stirred overnight at ambient temperature. The mixture is continuously extracted for 48 hours with ethyl acetate. The extracts are dried ($MgSO_4$), filtered, and the dried solution is treated with etheral HCl to give the desired product as a dihydrochloride salt.

EXAMPLE 5

3,3'-Thio bis-1-propanamine sulfone

3,3'-Thio bis-1-propanamine sulfoxide (3.76 g, 0.02 mole) is dissolved in chloroform (1 l) and the solution chilled in an ice bath. A solution of M-chloroperbenzoic acid (4.2 g of 85% - 0.02 mole) in chloroform (200 ml) is added dropwise. The mixture stirred for 1 hour at ice bath temperature, then extracted with aq. $Na_2CO_3$. The organic layer is evaporated, the residue taken up in ethanol and the product is precipitated as the dihydrochloride salt by the addition of excess etheral HCl.

EXAMPLE 6

N,N"-Thio bis(3,1-propanediyl)bis (1,3-propanediamine) sulfoxide

Step A: N,N'-bis(2-cyanoethyl)-3-aminopropyl sulfide:

3,3'-Thio bis-1-propanamine (14.8 g, 0.1 mole) is dissolved in ethanol (1.2 l) and acrylonitrile (11.3 g, 0.21 mole) is added dropwise. The mixture is stirred for 20 hours at ambient temperature and the resulting mixture is evaporated. The residue is distilled on a Kugelrohr apparatus to give the desired bis-cyanoethyl compound.

Step B: Lithium aluminum hydride (2.2 g, 0.06 mole) was added in portions to a solution of $AlCl_3$ (16 g, 0.12 mole) in anhydrous ether (700 ml). The 3 bis-cyanoethyl compound of Step A (5.08 g, 0.02 mole) is added dropwise over approximately 10 minutes and stirred overnight. The reaction mixture is treated dropwise with 15 ml of ethyl acetate, then 5 ml $H_2O$, followed by 50 ml of 15% NaOH. The resulting mixture is stirred until no gray color remains (24 hours), filtered, and the precipitate is washed with tetrahydrofuran. The filtrate is evaporated and the residue is dissolved in ethanol. The desired product is isolated as the tetrahydrochloride salt by addition of excess HCl gas to the solution and filtering off the precipitate.

EXAMPLE 7

N,N"-Thio bis(3,1-propanediyl)BIS(1,3-propandiamine) sulfoxide

N,N"-Thio bis (3,1 propanediyl)bis(1,3-propanediamine (2.6 g, 0.01 mole) is dissolved in a mixture of water (50 ml) and ethanol (5 ml). Sodium metaperiodate (2.3 g, 0.011 mole) is added and the mixture was stirred for 3 hours at ambient temperature. The mixture was continuously extracted with ethylacetate for 48 hours.

The ethylacetate solution was evaporated. The residue was redissolved in ethanol and the product is isolated as the tetrahydrochloride salt by addition of anhydrous HCl to the ethanol solution and filtering off the precipitate.

EXAMPLE 8

N,N"-Thio bis (3,1-propanediyl)bis(1,3-propanediamine) sulfone

The compound of Example 7 (1.3 g, 5 mmole) is dissolved in chloroform (700 ml). A solution of m-chloroperbenzoic acid (1.1 g of 85% peracid = 5 mmole) in chloroform (100 ml) is added dropwise. The reaction mixture was stirred for 18 hours; extracted with ag. $Na_2CO_3$, and evaporated. The residue is dissolved in ethanol and the product is precipitated as the tetrahydrochloride salt by addition of anhydrous HCl to the solution and filtering off the precipitate.

EXAMPLE 9

N,N'-bis-(Aminopropyl)-3,3'-oxy bis propaneamine·4CHl.

Step A: 3,3'-Oxybis-1-propaneamine•2HCl 11.89 ml (12.4 gm, 0.1 mole) of 3,3'-oxypropionitrile is combined with 150 ml acitic acid 29.2 ml of 12 N hydrochloric acid and 0.5 gm of Platinum oxide. Treat the resulting mixture with $H_2$ at 45 lb/in$^2$ until no more $H_2$ is taken up. Remove catalyst by filtration and evaporate at reduced pressure. Recrystallize from 50 ml methanol to obtain 4.5 gm (22%) of the desired product of this step as a white solid with mp<300°C.

Step B: N,N'-bis-cyanoethyl-oxy-bis-1-propaneamine 4.1 gm (0.02 mol) of the product of Step A is combined with 403 ml of EtOH, 2.63 ml (2.1 gm 0.04 mol) of acrylonitrile and 1.58 gm (0.0395 mol) of sodium hydroxide, stopper the vessel and allow to stir at room temperature for 3 days.

The mixture is then filtered and the solvent removed at reduced pressure to yield the desired product by distillation of the resulting residue. 4.1 gm (66%) b.p. 145° C at 0.1 mm.

<u>Step C:</u> 4.1 gm (0.017 mol) of the product of Step B is combined with 40 ml acetic acid, 8.5 ml 12 N hydrochloric acid and 0.4 gm Platinum oxide, treat with hydrogen at 45 $lb/in^2$ until no more $H_2$ is taken up. The catalyst is removed by filtration and the solvent removed at reduced pressure and the product recrystallized from EtOH to obtain 2.5 gm of the desired product of this example, mp. 274-278° C.

## <u>EXAMPLE 10</u>

<u>$N^1$,$N^8$-bis(3-aminopropyl)spermidine 5HCl</u>

<u>Step A:</u> $N_1$,$N^8$-bis cyanoethyl-4N-benzyl spermidine. $N^4$-Benzylspermidine, 4.7 gm (0.02 mol) prepared by this method of BERGERON JOC <u>45</u> 1589-92 (1980) is combined with 40 ml of ethanol and 2.63 ml (2.1 gm 0.04 mol) of acrylonitrile allow to stir overnight at room temperature. The solvent is removed at reduced pressure and the desired product of this step is obtained by distillation at 0.1 mm.

<u>Step B:</u> Combine 4.5 gm (0.0128 mol) of the product of Step A is combined with 100 ml of acetic acid, 10.7 ml of 12 N hydrochloric acid and 0.5 gm of $PtO_2$ treat with hydrogen at 45 $lb/in^2$ until no more $H_2$ is taken up. The catalyst is removed by filtration and the solvent is removed at reduced pressure. The desired product of this example is obtained by recrystallization from methanol.

CLAIMS FOR ALL THE DESIGNATED STATES EXCEPT AUSTRIA

1. A polyamine of the formula

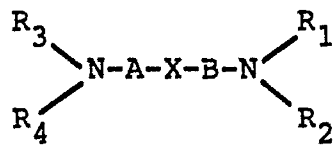

and the pharmaceutically acceptable acid addition salts thereof,
wherein X is $-CH_2-$, NR, O, S, SO or $SO_2$,
R is hydrogen, $(C_1-C_6)$alkyl, benzyl, mono- or di-substituted benzyl wherein the substituent is selected from hydroxy, halogen, $(C_1-C_4)$alkyl and $(C_1-C_4)$alkoxy,

$R_1$ and $R_3$ each are independently $-\overset{O}{\overset{\|}{C}}-(C_1-C_6)$alkyl, $-\overset{O}{\overset{\|}{C}}-$phenyl, $(C_1-C_6)$alkyl, hydrogen or $-(CH_2)_3NH_2$
$R_2$ and $R_4$ are $(C_1-C_6)$alkyl or hydrogen,
A is a $(C_3-C_4)$alkylene, optionally substituted with a $(C_1-C_4)$alkyl, and
B is a $(C_2-C_4)$alkylene, optionally substituted with a $(C_1-C_4)$alkyl, for use as a medicine.

2. N,N'-bis(3-aminopropyl)-1,8-octanediamine for use as a medicine.

3. Product containing a polyamine compound according to claim 1, and an ornithine dicarboxylase inhibitor and/or a S-adenosylmethionine dicarboxylase inhibitor as

a combined preparation for simultaneous, separate or sequential use as a medicine.

4. Product containing a polyamine compound according to claim 1, an ornithine decarboxylase inhibitor and/or interferon as a combined preparation for simultaneous, separate or sequential use as a medicine.

5. Product containing a polyamine compound according to claim 1, an ornithine decarboxylase inhibitor and/or a cytotoxic agent is a combined preparation for simultaneous, separate or sequential use as a medicine.

6. Product containing a polyamine compound according to claim 1, an ornithine decarboxylase inhibitor, interferon and a cytotoxic agent as a combined preparation for simultaneous, separate or sequential use as a medicine.

7. A compound according to claim 1, or a polyamine compound in a product according to claims 3, 4, 5 or 6 which is norspermidine.

8. A polyamine compound in a product according to claims 3, 4, 5 or 6, which is N,N'-bis(3-aminopropyl)-1,8-octanediamine.

9. A product according to claims 3, 4, 5 or 6 wherein the ornithine decarboxylase inhibitor is selected from the group consisting of α-difluoromethyl ornithine,

αmonofluoromethyl ornithine, (2R, 5R)-6-heptyn-2,5-diamine, and the methyl or ethyl ester of monofluoromethyl dehydrornithine.

10. A product according to claim 3, 4, 5 or 6 wherein the ornithine decarboxylase inhibitor is a α-difluoromethyl ornithine.

11. A compound according to claim 1 or a product according to claim 3, 4, 5, 6, 7, 8, 9 or 10 for the treatment of leukemia.

12. A compound according to claim 1 or a product according to claim 3, 4, 5, 6, 7, 8, 9 or 10 for the treatment of melanomas.

13. A compound according to claim 1 or a product according to claims 2, 3, 4, 5, 6, 7, 8 or 9 for the treatment of carcinomas.

14. A compound according to claim 1 or a product according to claim 3, 4, 5, 6, 7, 8, 9 or 10 for the treatment of Hodgkin's disease.

15. A compound according to claim 1 or a product according to claim 3, 4, 5, 6, 7, 8, 9 or 10 for the treatment of a leukemia of the group consisting of acute lymphoblastic, chronic lymphocytic, acute myloblastic and chronic myloblastic leukemia.

16. A compound according to claim 1 or a product according to claim 3, 4, 5, 6, 7, 8, 9 or 10 for the treatment of melanomas wherein the melanoma is either amelanotic or melanotic.

17. A compound according to claim 1 or a product according to claim 3, 4, 5, 6, 7, 8, 9 or 10 for the treatment of carcinomas wherein the carcinoma is a prostatic, lung, breast or colon carcinoma.

18. A compound of claim 1 which is norspermidine for the treatment of a neoplastic disease consisting of leukemia, melanoma, carcinoma, sarcoma and lymphoma.

19. A product according to claim 3 wherein the polyamine is norspermidine and the ornithine decarboxylase inhibitor is selected from a α-difluoromethyl ornithine, (2R, 5R)-6-heptyn--2,5-diamine and the methyl or ethyl ester of monofluoromethyl dehydrornithine for the treatment of a neoplastic disease consisting of leukemia, melanoma, carcinoma, sarcoma, and lymphoma.

20. A product according to claim 3 wherein the polyamine is norspermidine, an ornithine decarboxylase inhibitor is selected from a α-difluoromethyl ornithine, (2R, 5R)-6-heptyne-2,5-diamine and the methyl or ethyl ester of monofluoromethyl dehydrornithine and/or interferon for the treatment of a neoplastic disease consisting of leukemia, melanoma, carcinoma, sarcoma, and lymphoma.

21. Use of a compound of claim 1 or 2 or a product according to claim 3, 4, 5, 6, 17, 18, or 19 for the manufacture of a medicament for the treatment of neoplastic diseases.

22. Use of a compound of claim 1 or 2 or a product according to claim 3, 4, 5, 6, 17, 18, or 19 for the manufacture of a medicament for the treatment of neoplastic diseases wherein the neoplastic disease consists of leukemia, melanoma, carcinoma, sarcoma, and lymphoma.

23. Pharmaceutical composition for use in the treatment of neoplastic diseases which includes a compound of claim 1 or 2 in admixture with a pharmaceutically acceptable carrier.

for Austria
für Österreich
pour l'Autriche

CLAIMS FOR THE DESIGNATED STATE AUSTRIA

1. Process for preparing a polyamine of the formula

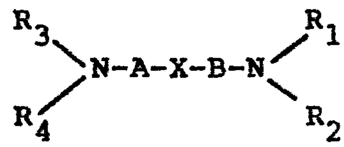

and the pharmaceutically acceptable acid addition salts thereof,
wherein X is $-CH_2-$, NR, O, S, SO or $SO_2$,
R is hydrogen, $(C_1-C_6)$alkyl, benzyl, mono- or di-substituted benzyl wherein the substituent is selected from hydroxy, halogen, $(C_1-C_4)$alkyl and $(C_1-C_4)$alkoxy,
$R_1$ and $R_3$ each are independently $-\overset{O}{\overset{\|}{C}}-(C_1-C_6)$alkyl, $-\overset{O}{\overset{\|}{C}}-$phenyl, $(C_1-C_6)$alkyl, hydrogen or $-(CH_2)_3NH_2$
$R_2$ and $R_4$ are $(C_1-C_6)$alkyl or hydrogen,
A is a $(C_3-C_4)$alkylene, optionally substituted with a $(C_1-C_4)$alkyl, and
B is a $(C_2-C_4)$alkylene, optionally substituted with a $(C_1-C_4)$alkyl, for use as a medicine.

2. Process according to claim 1 for preparing a polyamine which is N,N'-bis(3-aminopropyl)-1,8-octanediamine for use as a medicine.

for Austria
für Österreich
pour l'Autriche

3. Process for preparing a product containing a polyamine compound according to claim 1, and an ornithine dicarboxylase inhibitor and/or a S-adenosylmethionine dicarboxylase inhibitor and optionally in the presence of interferon and/or a cytotoxic agent as a combined preparation for simultaneous, separate or sequential use as a medicine.

4. Process according to claim 3 for preparing a product containing a polyamine compound according to claim 1, or 2 an ornithine decarboxylase inhibitor, interferon and a cytotoxic agent as a combined preparation for simultaneous, separate or sequential use as a medicine.

5. Process according to claim 1 or 3 for preparing a compound according to claim 1, or 2 or a polyamine compound in a product according to claim 3 which is norspermidine.

6. Process according to claim 3 for preparing a product wherein the ornithine decarboxylase inhibitor is selected from the group consisting of α-difluoromethyl ornithine, αmonofluoromethyl ornithine, (2R, 5R)-6-heptyn-2,5-diamine, and the methyl or ethyl ester of monofluoromethyl dehydrornithine.

7. Process according to claim 3 for preparing a product wherein the ornithine decarboxylase inhibitor is α-difluoromethyl ornithine.

8. Process for preparing a compound according to claim 1 or 2 or a product according to claim 3 for the manufacture of a medicament for the treatment of neoplastic diseases.

9. Process for preparing a compound of claim 1 or 2 or a product according to claim 3, for the manufacture of a medicament for the treatment of neoplastic diseases wherein the neoplastic disease consists of leukemia, melanoma, carcinoma, sarcoma, and lymphoma.

10. Process for preparing a pharmaceutical composition for use in the treatment of neoplastic diseases which includes a compound of claim 1, 2 or a product of claim 3 in admixture with a pharmaceutically acceptable carrier.